# Europäisches Patentamt
# European Patent Office
# Office européen des brevets

(19)

(11) Veröffentlichungsnummer: **0 047 514**
**A1**

(12)

# EUROPÄISCHE PATENTANMELDUNG

(21) Anmeldenummer: **81106968.1**

(22) Anmeldetag: **04.09.81**

(51) Int. Cl.³: **C 07 C 69/716, C 07 C 67/36**

(30) Priorität: **04.09.80 CH 6664/80**

(43) Veröffentlichungstag der Anmeldung: **17.03.82**
**Patentblatt 82/11**

(84) Benannte Vertragsstaaten: **AT BE CH DE FR GB IT LI LU NL SE**

(71) Anmelder: **LONZA AG, Gampel/Wallis (CH)**

(72) Erfinder: **Boosen, Karl-Josef, Dr. Dipl.-Chem.,
Längereben 206, CH-Erlach (Kanton Bern) (CH)**
Erfinder: **Fuchs, Rudolf, Dr., Rue des Saares 87,
CH-Neuchatel (CH)**

(74) Vertreter: **Von Füner, Alexander, Dr. K. L. Schiff Dr. A. v.
Füner et al, Dipl. Ing. P. Strehl Dr. U. Schübel-Hopf Dipl.
Ing. D. Ebbinghaus Dr. Ing. D. Finck Patentanwälte
Mariahilfplatz 2&3, D-8000 München 90 (DE)**

(54) Verfahren zur Herstellung von Acetondicarbonsäureestern.

(57) Verfahren zur Herstellung von Acetondicarbonsäureestern durch Carbonylierung von γ-Halogenacetessig-
ester.

EP 0 047 514 A1

# VERFAHREN ZUR HERSTELLUNG VON ACETONDICARBONSÄUREESTERN

## Beschreibung

Die Erfindung betrifft ein Verfahren zur Herstellung von Acetondicarbonsäureestern.

Es sind verschiedene Verfahren zur Herstellung von Acetondicarbonsäuren bekannt. Technische Bedeutung hat bisher allein die Herstellung aus Zitronensäure, z.B. durch Umsatz mit Oleum, erlangt (R. Adams et al., Org. Synth. Coll. $\underline{1,9}$ (1932)) (Vol. 1,10). Der Nachteil dieses Verfahrens liegt darin, daß große Mengen Abfallschwefelsäure anfallen.

Weitere Möglichkeiten, die keine technische Bedeutung erlangten, sind die Umsetzung von Aceton mit Kohlendioxid (DE-OS 24 29 627), die Oxidation von Zitronensäure mit Chlorsulfonsäure (DE-PS 11 60 841) und die Reaktion von Keten mit Phosgen (CH-PS 543 474).

Ziel der Erfindung ist ein neues, technisch anwendbares Verfahren zur Herstellung von Acetondicarbonsäureestern.

Erfindungsgemäß wird dies dadurch erreicht, daß man $\gamma$-Halogenacetessigester in Gegenwart einer Base und eines Alkohols car-

- 2 -

0047514

bonyliert.

Die Carbonylierung (Umsetzung mit CO) wird im geschlossenen Gefäß durchgeführt. Der anzuwendende CO-Druck liegt zweckmäßig bei 20 bis 150 bar, vorzugsweise bei 50 bis 100 bar.

Die anzuwendende Temperatur liegt vorteilhaft bei 20 bis $100^\circ C$, vorzugsweise bei 40 bis $80^\circ C$.

Die Carbonylierung wird nach bekannter Weise in Gegenwart von Carbonylierungskatalysatoren, nämlich Metallcarbonylen, wie $Fe(CO)_5$, $Ni(CO)_4$, vorzugsweise $Co_2(CO)_8$, durchgeführt.

Die Menge an anzuwendendem Katalysator liegt vorteilhaft bei 2 bis 10 Mol-% pro Mol $\gamma$-Halogenacetessigester.

Als $\gamma$-Halogenacetessigester kommen $\gamma$-Chlor-, $\gamma$-Fluor- und $\gamma$-Bromacetessigester in Frage. Vorzugsweise werden $\gamma$-Chloracetessigester angewendet. Der Esterrest richtet sich danach, welcher Acetondicarbonsäureester erhalten werden soll. Ebenso danach richtet sich die Art des anzuwendenden Alkohols. Vorzugsweise wird der Alkohol eingesetzt, der dem Esterrest des $\gamma$-Halogenacetessigesters entspricht. Pro Mol $\gamma$-Halogenacetessigester werden vorteilhaft 5 bis 15 Mol Alkohol angewendet.

Als Base kommen die Hydroxide, Carbonate und Phosphate der Alkalimetalle wie $K_2CO_3$, $Na_2CO_3$, $NaHCO_3$, $K_3PO_4$, $Na_3PO_4$, $Na_2HPO_4$, in Betracht. Vorzugsweise werden Alkalicarbonate verwendet.

Die Basen kommen in Mengen von 0,8 bis 1,2 Mol pro Mol $\gamma$-Halogenacetessigester zum Einsatz.

Beispiel 1

In einem 300 ml-Autoklaven werden 120 ml $C_2H_5OH$, 35,4 g $ClCH_2COCH_2COOC_2H_5$, 16,6 g $K_2CO_3$ und 3 g $Co_2(CO)_8$ unter 100 bar CO 7 Stunden auf $65^\circ C$ erhitzt. Nach Abdampfen des $C_2H_5OH$

werden zum Rückstand 100 ml $CH_2Cl_2$ und 70 ml $H_2O$ gegeben und die Lösung mit 5 ml 2n HCl neutralisiert. Die organische Phase wird abgetrennt und destilliert.

Ausbeute: 24,3 g Acetondicarbonsäurediäthylester, (Kp. 0,13 70-75$^O$C) = 59,9 %.

Reinheit: 99,6 %.

<u>Beispiele 2 bis 10</u>

| Nr. | $\gamma$-Ha-logen | Ester | Druck bar | Alkohol | Base | Kat. | Reaktion Zeit h | Temp. $^{\circ}C$ | Aus-beute % |
|---|---|---|---|---|---|---|---|---|---|
| 2 | Cl | $C_2H_5-$ | 90 | $C_2H_5OH$ | $K_2CO_3$ | $Co_2(CO)_8$ | 7 | 65 | 70 |
| 3 | Cl | $CH_3-$ | 100 | $CH_3OH$ | $K_2CO_3$ | $Co_2(CO)_8$ | 7 | 65 | 31 |
| 4 | Cl | $C_2H_5-$ | 80 | $C_2H_5OH$ | $Na_2CO_3$ | $Co_2(CO)_8$ | 6 | 80 | 35 |
| 5 | Br | $C_2H_5-$ | 60 | $C_2H_5OH$ | $K_2CO_3$ | $Co_2(CO)_8$ | 7 | 65 | 58 |
| 6 | Cl | $C_2H_5-$ | 90 | $C_2H_5OH$ | $Na_2HPO_4$ | $Fe(CO)_5$ | 6 | 70 | 52 |
| 7 | Br | $CH_3-$ | 90 | $CH_3OH$ | $NaHCO_3$ | $Fe(CO)_5$ | 6 | 70 | 44 |
| 8 | Cl | $C_2H_5-$ | 90 | $C_2H_5OH$ | $K_2CO_3$ | $Ni(CO)_4$ | 7 | 65 | 62 |
| 9 | Cl | $C_2H_5-$ | 90 | $C_2H_5OH$ | $NaOH$ | $Co_2(CO)_8$ | 7 | 70 | 21 |
| 10 | Cl | $C_2H_5-$ | 150 | $C_2H_5OH$ | $K_2CO_3$ | $Co_2(CO)_8$ | 7 | 55 | 59 |

Eine weitere Ausführungsform besteht darin, daß man vor dem Abdampfen des Alkohols die anorganischen Salze abfiltriert.

Anstelle von $CH_2Cl_2$ können auch andere Lösungsmittel, wie z.B. $CHCl_3$ oder Toluol eingesetzt werden.

Die Reinheiten der erzielten Produkte liegen zwischen 98,2 % und 99,7 %.

Als Alkohole kommen im wesentlichen niedere aliphatische Alkohole, insbesondere solche mit 1 - 5 Kohlenstoffatomen in Frage.

Patentansprüche

1. Verfahren zur Herstellung von Acetondicarbonsäureestern, d a d u r c h   g e k e n n z e i c h n e t , daß man $\gamma$-Halogenacetessigester in Gegenwart eines basischen Mittels und eines Alkohols carbonyliert.

2. Verfahren nach Anspruch 1, d a d u r c h   g e - k e n n z e i c h n e t , daß man die Carbonylierung mit Hilfe von $Co_2(CO)_8$ als Katalysator durchführt.

3. Verfahren nach Anspruch 1 und 2, d a d u r c h   g e - k e n n z e i c h n e t , daß man als $\gamma$-Halogenacetessig- ester den $\gamma$-Chloracetessigester verwendet.

4. Verfahren nach Anspruch 1 bis 3 , d a d u r c h   g e - k e n n z e i c h n e t , daß man als Basen Carbonate, Hydrogencarbonate oder Phosphate der Alkalimetalle verwendet.

5. Verfahren nach Anspruch 1 bis 4, d a d u r c h   g e - k e n n z e i c h n e t , daß man als Alkohol den Alkohol verwendet, der dem Esterrest des $\gamma$-Halogenacetessigesters entspricht.

| Kategorie | Kennzeichnung des Dokuments mit Angabe, soweit erforderlich, der maßgeblichen Teile | betrifft Anspruch |
|---|---|---|
| | DE - A1 - 2 553 931 (DYNAMIT NOBEL AG)<br><br>* Beispiele *<br><br>---- | 1,2 |

**EINSCHLÄGIGE DOKUMENTE**

**KLASSIFIKATION DER ANMELDUNG (Int. Cl.³)**

C 07 C 69/716

C 07 C 67/36

**RECHERCHIERTE SACHGEBIETE (Int. Cl.³)**

C 07 c 69/00

C 07 C 67/00

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| WIEN | 04-11-1981 | HOFBAUER |

EPA form 1503.1  06.78